Europäisches Patentamt

European Patent Office

Office européen des brevets

(11) Numéro de publication: **0 227 506**
**A1**

(12) # DEMANDE DE BREVET EUROPEEN

(21) Numéro de dépôt: 86402452.6

(22) Date de dépôt: 03.11.86

(51) Int Cl.⁴: **C 12 P 21/02**
C 07 K 5/06

(30) Priorité: 05.11.85 FR 8516366

(43) Date de publication de la demande:
01.07.87 Bulletin 87/27

(84) Etats contractants désignés: DE GB NL

(71) Demandeur: SOCIETE FRANCAISE HOECHST Société
anonyme dite:
3, avenue du Général de Gaulle
F-92800 Puteaux (FR)

(72) Inventeur: Monsan, Pierre
Renoufail Mondonville
F-31700 Blagnac (FR)

Paul, François
28 rue du Vivier
F-31650 Saint Orens de Gameville (FR)

Duchiron, Francis
58 Chemin de Nicol
F-31200 Toulouse (FR)

(74) Mandataire: Rinuy, Santarelli
14, avenue de la Grande Armée
F-75017 Paris (FR)

(54) Procédé biologique de préparation de N-(N-benzyloxycarbonyl L-aspartyl-1) L-phénylalaninate de méthyle.

(57) Ce procédé impliquant l'action d'une protéase sur un mélange d'acide L-N-benzyloxycarbonyl aspartique et de DL-phenylalaninate de méthyle est caractérisé par le fait que la protéase est la protéase protéolytique exocellulaire neutre extraite d'une culture de Micrococcus caseolyticus.

EP 0 227 506 A1

## Description

Procédé biologique de préparation de N-(N-benzyloxycarbonyl L-aspartyl-l) L-phénylalaninate de méthyle.

La présente invention concerne un procédé biologique de préparation de N-(N-benzyloxycarbonyl L-aspartyl-1) L-phénylalaninate de méthyle.

Le N-(N-benzyloxycarbonyl L-aspartyl-1) L-phényl-alaninate de méthyle, désigné ci-après NZAPMe, largement décrit dans la littérature, est une matière première précieuse pour accéder au N-(L-aspartyl-1) L-phénylalaninate de méthyle, communément appelé aspartame, doué de propriétés édulcorantes remarquables (brevet des Etats-Unis d'Amérique N 3 492 131 et R.H. Mazur et al., J. Amer. Chem. Soc., 1969, 91, 2684).

On sait accéder au NZAPMe par des procédés soit chimiques, soit biologiques.

Les très nombreux procédés chimiques connus font généralement intervenir une condensation peptidique classique entre les deux acides aminés constitutifs, associée éventuellement à une succession de blocage et de déblocage de certains de leurs sites réactifs, de manière à atteindre la configuration souhaitée (brevet français N 2 091 628).

L'obtention par voie biologique du NZAPMe est connue par D.D. Petkov et al. , Tetrahedron Letters, 1984, 25(34) 3751 ; J. Isowa et al., Tetrahedron Letters, 1979, 2611 : K. Oyama et al., J. Org. Chem., 1981, 46, 5241 et J. Chem. Soc. (B), 1981, 356 : les brevets français N 2 328 695, 2 378 747 et 2 453 137 : les brevets des Etats-Unis d'Amérique N 4 129 945 et 4 129 946 : les demandes de brevet européen N 0 075 160 et 0 099 585 et les demandes de brevet japonais N 80(55)-19051 et 80(55)-74492.

Selon cet état de la technique, on accède au NZAPMe en faisant réagir, à un pH convenable et généralement dans un milieu aqueux tamponné, une métalloprotéinase sur un mélange d'acide N-benzyloxycarbonyl aspartique, racémique ou lévogyre désigné ci-après DL- ou L-ZAspOH et de phénylalaninate de méthyle racémique ou lévogyre, désigné ci-après DL- ou L-HPhOMe. Les métalloprotéinases utilisées possèdent un ion métallique dans le centre actif et sont des enzymes provenant de micro-organismes tels que des protéases neutres de ray fungus, Prolisine, Thermolycine, Collagénase, Cortulus atroxprotéase. Il est également possible d'utiliser des enzymes bruts tels que Thermoase, Tacynase-N. Pronase ou des protéases issues de souches de Bacillus, Streptomyces, Pseudomonas telles que la souche de Bacillus N 36 Ferm P-4736 ou de certains champignons notamment des Aspergillus.

Par ailleurs, on sait que sous certaines conditions de culture, le micro-organisme Micrococcus caseolyticus, isolé dans du lait de vache, produit un système protéolytique, désigné ci-après SP, d'où l'on peut extraire une protéase neutre, unique, protéolytique et exocellulaire, désignée ci-après P, de poids moléculaire compris entre 35000 et 41000, présentant la presque totalité de l'activité protéolytique (M. Desmazeaud et al., Ann. Bio. Anim. Biochem. Biophys., 1968, 8, 419-429 et 565-577 et European J. Biochem. 1971, 19, 51-55).

Or la Demanderesse a découvert avec étonnement que cette protéase P fournit économiquement, facilement et avec de bons rendements du NZAPMe lorsqu'on la fait réagir sous certaines conditions sur un mélange de L-ZAspOH et de DL-HPhOMe.

La présente invention a donc pour objet un procédé de préparation biologique de NZAPMe par action d'une protéase sur un mélange de L-ZAspOH et de DL-HPhOMe caractérisé en ce que la protéase est la protéase protéolytique exocellulaire neutre extraite d'une culture de Micrococcus caseolyticus.

La protéase neutre protéolytique exocellulaire, P, est obtenue à partir du surnageant de culture à pH 7,0 de la souche de Micrococcus caseolyticus déposée dans la Collection Nationale de Culture de Micro-organisme à l'Institut Pasteur à Paris sous le N 1194 le 28 avril 1982.

Après élimination des bactéries par centrifugation, ce surnageant est traité par du sulfate d'ammonium puis le précipité obtenu est dissous dans une solution aqueuse de chlorure de sodium et ensuite cette solution est soumise à une ultrafiltration puis à une lyophilisation. On isole ainsi une poudre constituée par un système protéolytique, désigné ci-après SP, contenant, entre autres, la protéase P. Cette protéase P est ensuite purifiée d'abord par chromatographie sur diéthylaminoéthyl-cellulose en exploitant la propriété que SP n'est pas retenu sur la colonne équilibrée avec du tampon tris-(hydroxyméthyl)-aminométhane (désigné TRIS)-maléate 50 mM alors qu'il est retenu lorsque la colonne est équilibrée en tampon TRIS-maléate 5 mM. Puis cette purification est terminée par une chromatographie sur gel (Sephadex G 100 commercialisé par Pharmacia-France S.A. à 78391 Bois d'Arcy, France) préparé en tampon TRIS-maléate 5 mM à pH 7,0.

Un exemple d'une telle préparation du système protéolytique, SP, est donné dans la demande de brevet français N 82.07883 publiée sous le N 2 525 865 et pour ce qui est de la protéase P par M. Desmazeaud et al., Ann. Biol. Anim. Bioch. Biophys., 1968, 8, 565-577.

Selon une variante du procédé de la présente invention, on peut remplacer avantageusement la protéase P par le système protéolytique SP tel que défini précédemment.

L'activité protéolytique soit de la protéase P, soit du système protéolytique SP est déterminée par mesure (variation de la densité optique à 280 nm) de la quantité de caséine hydrolysée soit par P, soit par SP (non précipitable par l'acide trichloracétique) dans des conditions standard.

Une unité d'activité protéolytique, désignée ci-après U, est définie comme la quantité d'enzyme qui, agissant dans les conditions du dosage, provoque une variation de la densité optique à 280 nm de 0,001 unité de densité optique par minute, à la température de 30 C.

La technique de préparation par voie biologique du NZAPMe étant bien connue en elle-même, les exemples donnés ci-après ne sont mentionnés que pour illustrer la substitution de la protéase utilisée dans ces procédés par soit le système protéolytique SP, soit la protéase P, extraits d'une culture de Micrococcus caseolyticus.

Habituellement, on fait réagir durant une à cent heures, à une température comprise entre 10 et 45°C et à un pH compris entre 4 et 8, la protéase P ou le système protéolytique SP sur une solution tamponnée de L-ZAspOH et de DL-HPhOMe, en présence éventuellement d'ions calcium. Au fur et à mesure de l'avancement de la condensation, le produit cherché précipite sous la forme d'un adduct connu 1:1 avec le D-HPhOMe qu'il suffit, en fin de réaction, d'isoler par filtration puis de transformer par des méthodes connues en soi en NZAPMe pur.

Selon le procédé de l'invention, la protéase P ou le système protéolytique SP condense sélectivement le L-ZAspOH sur le L-HPhOMe si bien que le procédé peut être mis en oeuvre au départ d'au moins deux moles de DL-HPhOMe par mole de L-ZAspOH engagé.

La condensation enzymatique est habituellement réalisée en solution aqueuse diluée, avantageusement en solution 0,05 à 1 molaire de L-ZAspOH, en présence de protéase P ou du système enzymatique SP, à raison de 500 à 100 000 U par mole de L-ZAspOH.

Le L-ZAspOH et le DL-HPhOMe sont des produits d'accès facile, largement décrits dans la littérature. Avantageusement, on utilise le chlorhydrate de DL-HPhOMe et dans ce cas, le milieu réactionnel est ajusté au pH désiré par de la soude 2N.

Les exemples suivants sont donnés à titre illustratif et nullement limitatif de l'invention.

Exemple 1

On abandonne 15 heures à 30°C une solution ajustée à pH = 6,7 contenant :
- 1 mmole (215 mg) de chlorhydrate de DL-phényl-alaninate de méthyle,
- 0,5 mmole (133,5 mg) d'acide L-N-benzyloxycarbonyl aspartique,
- 25 000 U d'enzyme P extrait du bouillon de culture de Micrococcus caseolyticus,
dans 5 ml d'une solution de tampon de chlorhydrate de TRIS 20 mM à pH 6,7 contenant du chlorure de calcium à la concentration 1.5 mM.

Au fur et à mesure de l'avancement de la condensation, il se forme un précipité que l'on filtre en fin de réaction. On isole ainsi 230 mg (0,375 mmole) de l'adduct 1:1 de N-(N-benzyloxycarbonyl L-aspartyl-1) L-phénylalaninate de méthyle et de D-phénylalaninate de méthyle qui fournit après traitement par 0,5 ml d'acide chlorhydrique 1N en suspension dans 5 volumes d'eau à 20°C, 161 mg soit 0,375mmole de N-(N-benzyloxycarbonyl L-aspartyl-1) L-phényl-alaninate de méthyle pur soit un rendement de 75 % de la théorie calculée par rapport à l'acide L-N-benzyloxycarbonyl aspartique mis en oeuvre.

Exemple 2

On reproduit l'exemple 1 en utilisant 14 000 U du système protéolytique SP au lieu de 25 000 U d'enzyme P. On obtient ainsi en fin de réaction du N-(N-benzyloxycarbonyl L-aspartyl-1) L-phénylalaninate de méthyle pur avec un rendement de 60 % de la théorie calculée par rapport à l'acide L-N-benzyloxycarbonyl aspartique mis en oeuvre.

Il va de soi que la présente invention n'a été décrite qu'à titre purement explicatif et nullement limitatif et que toute modification, notamment au niveau des équivalences, pourra y être apportée sans toutefois sortir de son cadre.

**Revendications**

1. Procédé de préparation biologique de N-(N-benzyloxycarbonyl L-aspartyl-1) L-phénylalaninate de méthyle par action d'une protéase sur un mélange d'acide L-N-benzyloxycarbonyl aspartique et de DL-phénylalaninate de méthyle caractérisé en ce que la protéase est la protéase protéolytique exocellulaire neutre extraite d'une culture de Micrococcus caseolyticus.

2. Procédé selon la revendication 1, caractérisé en ce que la protéase protéolytique exocellulaire neutre est mise en oeuvre sous la forme du système protéolytique extrait d'une culture de Micrococcus caseolyticus.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 124 313 (AJINOMOTO) <br> * En entier * <br><br> --- | 1,2 | C 12 P 21/02 <br> C 07 K 5/06 |
| X | EP-A-0 154 472 (AJINOMOTO) <br> * En entier * <br><br> --- | 1,2 | |
| D,Y | EP-A-0 099 585 (AJINOMOTO) <br> * En entier * <br><br> --- | 1,2 | |
| D,X <br> Y | FR-A-2 378 747 (TOYO SODA) <br><br> * En entier * <br><br> --- | 1,2 | |
| D,X <br> Y | FR-A-2 453 137 (SAGAMI) <br><br> * En entier * <br><br> --- | 1,2 | **DOMAINES TECHNIQUES RECHERCHES (Int. Cl.4)** |
| Y | BIO/TECHNOLOGY, vol. 3, mai 1985, pages 459-464; K. NAKANISHI et al.: "Continuous synthesis of N-(benzyloxycarbonyl)-L-aspartyl-L-phenylalanine methyl ester with immobilized thermolysin in an organic solvent" <br> * En entier * <br><br> ---     -/- | 1 | C 12 P 21/00 <br> C 07 K 5/00 |

Le présent rapport de recherche a été établi pour toutes les revendications

| Lieu de la recherche | Date d'achèvement de la recherche | Examinateur |
|---|---|---|
| LA HAYE | 10-02-1987 | RAJIC M. |

## RAPPORT DE RECHERCHE EUROPEENNE

Office européen
des brevets

Numero de la demande

EP 86 40 2452

Page 2

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl.4) |
|---|---|---|---|
| X,Y | CHEMICAL ABSTRACTS, vol. 104, 1986, page 559, résumé no. 167195v, Columbus, Ohio, US; & BE-A-902 474 (FARMITALIA CARLO ERBA SpA) 16-09-1985 * Résumé * | 1,2 | |
| X,Y | CHEMICAL ABSTRACTS, vol. 102, 1985, page 441, résumé no. 44264t, Columbus, Ohio, US; K. OYAMA: "Syntheses of peptides by proteolytic enzymes", & YUKAGAKU 1984, 33(10), 697-702 * Résumé * | 1,2 | |
| Y | CHEMICAL ABSTRACTS, vol. 103, 1985, page 237, résumé no. 2502j, Columbus, Ohio, US; H. JAKUBKE et al.: "Basic principles of protease-catalyzed joining of peptide bonds", & ANGEW. CHEM. 1985, 97(2), 79-87 * Résumé * | 1 | DOMAINES TECHNIQUES RECHERCHES (Int. Cl.4) |
| Y | CHEMICAL ABSTRACTS, vol. 103, 1985, page 352, résumé no. 174726b, Columbus, Ohio, US; K. OYAMA: "Syntheses of peptides by proteolytic enzymes", & TOYO SODA KENKYU HOKOKU 1985, 29(2), 145-51 * Résumé * | 1 | |

Le présent rapport de recherche a été établi pour toutes les revendications

| Lieu de la recherche | Date d'achèvement de la recherche | Examinateur |
|---|---|---|
| LA HAYE | 10-02-1987 | RAJIC M. |